# FASCICULE DE BREVET EUROPEEN

(11) **EP 4 434 565 B1**
(45) Date de publication et mention de la délivrance du brevet: **30.07.2025**
(21) Numéro de dépôt: 24161484.1
(22) Date de dépôt: 05.03.2024
(51) Int. Cl.: A61M 16/00, A61M 16/10, A61M 16/12, A61M 16/20, G01N 33/00

(54) **DISPOSITIF DE CALIBRATION DES CAPTEURS D'UN APPAREIL DE DÉLIVRANCE DE NO**
VORRICHTUNG ZUR KALIBRIERUNG VON SENSOREN EINER NO-AUSGABEVORRICHTUNG
DEVICE FOR CALIBRATING THE SENSORS OF A NO-DELIVERY DEVICE

(30) Priorité: 21.03.2023 FR 2302598
(43) Date de publication de la demande: 25.09.2024
(73) Titulaire: INOSYSTEMS, 92160 Antony (FR)
(72) Inventeur: BOULANGER, Thierry, 92160 Antony (FR); MARCHAL, Frédéric, 92160 Antony (FR); PATEL, Akshar, 92160 Antony (FR)
(74) Mandataire: Air Liquide

(56) Documents cités:
- EP-A1- 2 522 384
- EP-A1- 2 581 103
- CN-A- 104 857 607
- US-A1- 2015 320 951

## Description

L'invention concerne un dispositif de calibration pour calibrer les moyens de mesure d'un appareil de délivrance de NO, en particulier les capteurs de NO, O₂ et NO₂ dudit appareil de délivrance de NO.

Le monoxyde d'azote inhalé ou NOi est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

Pour mettre en œuvre une thérapie par NO inhalé, on utilise classiquement une installation de fourniture de gaz, aussi appelée installation d'administration de NO, comprenant un appareil de délivrance de NO, un ventilateur médical, c'est-à-dire un appareil d'assistance respiratoire, et un circuit patient.

L'appareil de délivrance de NO permet d'injecter un mélange gazeux à base de NO, typiquement un mélange NO/azote, dans le circuit patient alimenté par ailleurs en un flux gazeux contenant de l'oxygène (environ 21% vol.), tel de l'air ou un mélange oxygène/azote, fourni par le ventilateur médical.

Le circuit patient comprend en général un ou plusieurs conduits flexibles reliés fluidiquement à une interface respiratoire, telle une sonde d'intubation trachéale ou analogue, servant à délivrer au patient à traiter, une dose donnée de NO, c'est-à-dire une posologie.

Une telle installation de fourniture de gaz est décrite par exemple par EP3821929. Ce type d'installation est utilisé en milieu hospitalier pour administrer le traitement par NOi et ainsi soigner les patients ayant besoin d'inhaler du NO pour traiter leur hypertension artérielle pulmonaire.

Afin de s'assurer que le mélange gazeux administré au patient contient les proportions désirées de NO et d'oxygène mais, à l'inverse, ne contient pas ou peu de NO₂, il convient de prélever régulièrement des échantillons gazeux dans le circuit patient, typiquement à proximité de l'interface respiratoire, et de les analyser.

Pour ce faire, l'appareil de délivrance de NO est relié fluidiquement, via une ligne de prélèvement de gaz, au circuit patient afin de permettre de prélever une portion du gaz qui y circule, typiquement 100 à 300 ml/min environ, afin de l'analyser et de vérifier si les teneurs en gaz sont conformes aux valeurs désirées, en particulier en NO, NO₂ et O₂.

EP2522384 propose un analyseur de gaz embarqué dans l'appareil de délivrance de NO servant à mesurer les teneurs en NO, NO₂ et O₂ dans les échantillons gazeux prélevés. Les moyens de mesure de concentration mettent en œuvre des capteurs de type électrochimique. Or, ce type de capteurs souffre d'une dérive au fil du temps et nécessite une calibration périodique. L'étalonnage à «zéro» des capteurs nécessite un mélange gazeux de référence dépourvu de NO et de NO₂ afin de déterminer leur réponse nominale au repos, notamment en prélevant l'air ambiant. Par ailleurs, la génération du point de calibration haut, par exemple à 40 ppmv de NO (le gain), se fait via une électrovanne tout ou rien venant connecter la source de NO (i.e. mélange de 800 ppmv de NO diluée dans N₂) à l'analyseur de gaz

Cependant, cette solution n'est pas idéale car le fait d'avoir recours à une électrovanne entre la source de NO et l'analyseur de gaz nécessite de modifier profondément l'architecture de l'appareil de délivrance de NO, en particulier engendre des changements notables de ses composants électroniques et mécaniques. Il n'est donc pas possible ou pas aisé d'intégrer cette solution aux appareils existants, c'est-à-dire déjà en service. De plus, une telle électrovanne, même fermée, peut être sujette à de faibles fuites et du NO de « fuite » peut venir se mélanger au gaz à analyser et provoquer des interférences majeures au niveau des capteurs.

Par ailleurs, on connait EP2581103 qui décrit un procédé de calibration d'un appareil de fourniture de NO, US2015/320951 qui enseigne un procédé permettant de prédire lorsqu'une bouteille de NO alimentant un appareil de fourniture de NO sera vide, et CN104857607 propose un dispositif de calibration de concentration en oxygène.

Partant de là, un problème est de permettre une calibration périodique efficace d'un appareil de délivrance de NO, de façon autonome et indépendamment de l'appareil de délivrance de NO, et ce, sur n'importe quel appareil de délivrance de NO, y compris ceux du parc existant, de préférence sans risque d'interférence avec l'analyseur de gaz de l'appareil de délivrance de NO considéré.

Une solution de l'invention concerne un dispositif de calibration pour calibrer les moyens de mesure d'un appareil de délivrance de NO, lesdits moyens de mesure comprenant un capteur de NO, un capteur de NO₂ et un capteur d'O₂, comprenant une ligne d'admission de gaz comprenant, agencés en série :
- une vanne pour contrôler la circulation de gaz dans la ligne d'admission de gaz,
- un régulateur de précision configuré pour délivrer une pression fixe prédéfinie dans la ligne d'admission de gaz,
- un dispositif à orifice calibré et
- un dispositif à venturi.

De plus, le dispositif à venturi comprend un corps principal définissant un volume interne et comprenant :
- un orifice d'entrée de gaz relié fluidiquement au dispositif à orifice calibré pour permettre une entrée de gaz provenant du dispositif à orifice calibré, dans le volume interne,
- un orifice d'admission de gaz relié fluidiquement à l'atmosphère pour permettre une entrée d'air dans le volume interne,
- un orifice d'échappement relié fluidiquement à l'atmosphère pour permettre une évacuation à l'atmosphère, d'une partie du gaz contenu dans le volume interne, et
- un port de sortie pour fournir au moins une partie du gaz contenu dans le volume interne à un appareil de délivrance de NO.

Selon le mode de réalisation considéré, le dispositif de calibration de l'invention peut comprendre l'une ou plusieurs des caractéristiques suivantes :
- la vanne est mobile entre au moins :
   ∘ une position de fermeture, i.e. vanne fermée, dans laquelle la circulation de gaz dans la ligne d'admission de gaz est empêchée,
   ∘ et une position d'ouverture, i.e. vanne ouverte, dans laquelle la circulation de gaz dans la ligne d'admission de gaz est autorisée.
- la vanne est une vanne manuelle actionnable par un utilisateur, i.e. actionnable entre au moins les deux positions.
- le régulateur de précision est configuré pour délivrer une pression fixe comprise entre 350 et 700 mbar.
- le dispositif à orifice calibré comprend un orifice de sortie de diamètre (non nul) inférieur à 500 µm.
- la ligne d'admission de gaz, la vanne manuelle, le régulateur de précision, le dispositif à orifice calibré et le dispositif à venturi sont agencés dans un boitier commun.
- l'orifice d'admission de gaz présente une aire de section comprise entre 0.5 et 10 mm².
- il comprend en outre des moyens de raccordements amont agencés au niveau d'un port d'entrée de la ligne d'admission de gaz, configurés pour y raccorder un conduit flexible.
- il comprend en outre des moyens de raccordement aval agencés au niveau du port de sortie du dispositif à venturi, configurés pour y raccorder une ligne d'alimentation en gaz d'un analyseur de gaz d'un appareil de délivrance de NO.
- le diamètre de sortie de l'orifice calibré et le diamètre de l'orifice d'admission venturi sont dimensionnés pour obtenir un rapport constant entre le débit sortant de l'orifice calibré et le débit entrant par l'orifice d'admission venturi compris entre 10 et 30.
- le diamètre de sortie de l'orifice calibré et le diamètre de l'orifice d'admission venturi sont dimensionnés pour obtenir un rapport constant sur une plage de pression donnée comprise entre 200 et 900mb par exemple.

L'invention concerne aussi une utilisation d'un dispositif de calibration selon l'invention, pour calibrer les moyens de mesure d'un appareil de délivrance de NO, lesdits moyens de mesure comprenant un capteur de NO, un capteur de NO₂ et un capteur d'O₂.

De préférence, le dispositif de calibration est raccordé fluidiquement à une (ou plusieurs) bouteille de gaz contenant un mélange NO/N₂ alimentant la ligne d'admission de gaz du dispositif de calibration, en amont de la vanne manuelle.

Avantageusement, la bouteille de gaz sous pression contient un mélange de NO/azote contenant de 100 à 2000 ppmv de NO dilué dans de l'azote (N₂), typiquement entre 200 et 1500 ppmv de NO, le reste étant de l'azote.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un dispositif de calibration selon la présente invention ;
Fig. 2 est un tableau de résultats illustrant des tests réalisés avec le dispositif de calibration de Fig. 1 ;
Fig. 3 schématise un mode de réalisation de l'analyseur de gaz d'un dispositif de délivrance de NO devant être périodiquement calibré ;
Fig. 4 schématise le dispositif de calibration de Fig. 1 associé au dispositif de délivrance de NO de Fig. 3, lors d'une procédure de calibration ;
Fig. 5 illustre les performances d'une pompe faisant partie de l'analyseur de gaz de Fig. 3 ; et
Fig. 6 schématise un mode de réalisation d'une installation de fourniture de gaz à un patient comprenant le dispositif de délivrance de NO de Fig. 3 et un ventilateur médical alimentant un circuit patient.

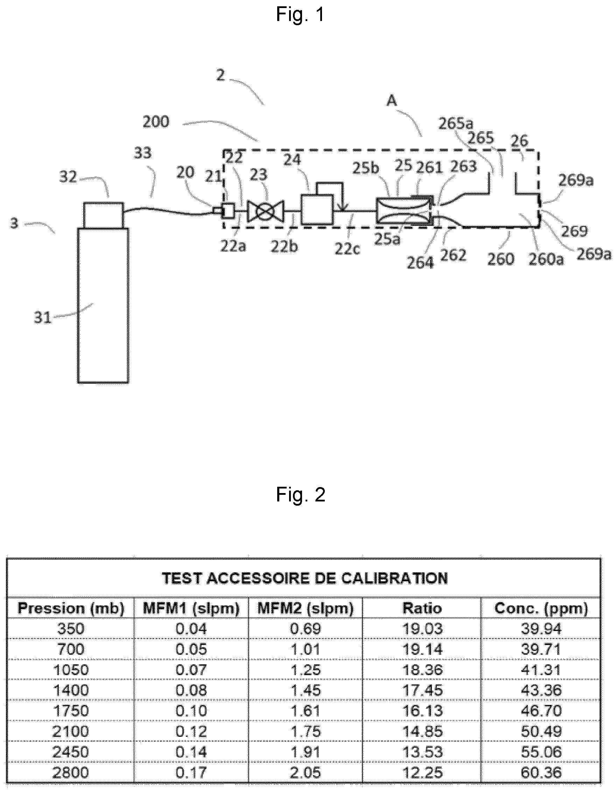

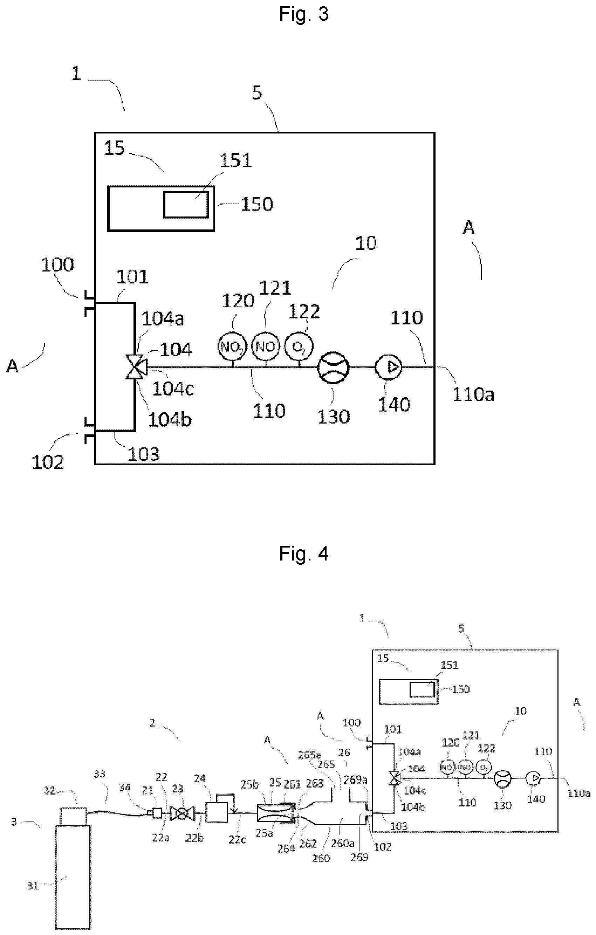

Fig. 1 schématise un dispositif de calibration 2, i.e. un système ou ensemble de calibration, selon l'invention, utilisable pour calibrer les capteurs d'un appareil de délivrance de NO, tel celui illustré sur Fig. 3, comme détaillé ci-après.

Sur Fig. 1, le dispositif de calibration 2 selon l'invention est raccordé, au niveau d'un port d'entrée 21, à un conduit flexible 33 qui est lui-même relié fluidiquement à une source de NO 3. Le raccordement se fait via des moyens de raccordement amont 20 situés au niveau du port d'entrée 21, tel un connecteur ou analogue, de préférence « à connexion rapide » qui permet à un utilisateur de pouvoir connecter et déconnecter le flexible de raccordement de manière simple et rapide.

La source de NO 3 est ici une bouteille de gaz sous pression 31 contenant typiquement un mélange de NO/azote, de préférence un mélange de NO/azote contenant de 100 à 2000 ppmv de NO dilué dans de l'azote, par exemple contenant ici environ 800 ppmv de NO dilué dans de l'azote (N₂). Le volume interne de la bouteille de gaz 31 est de préférence compris entre 2 et 20L (équivalent en eau). Le mélange NO/N₂ y est stocké à une pression d'au moins 150 bar, de préférence au moins 180 bar, par exemple de l'ordre de 200 bar ou plus, lorsqu'elle est pleine, c'est-à-dire avant tout soutirage de gaz.

La bouteille de gaz 31 est surmontée d'un robinet-détendeur 32 (ou RDI) qui permet d'abaisser de contrôler le débit de gaz et de diminuer la pression du gaz provenant de la bouteille 31 jusqu'à une pression de détente fixe donnée, par exemple entre 3 et 7 bar, par exemple de l'ordre de 4 ou 5 bar.

Le port d'entrée 21 du dispositif de calibration 2 met en relation fluidique le flexible de raccordement 33 alimenté par le gaz provenant de la bouteille 31 à une ligne d'admission de gaz 22 sur laquelle sont agencés différents composants, qui sont successivement traversé par le gaz, à savoir le mélange NO/N₂ provenant de la bouteille de gaz sous pression 31. Le gaz y circule dans un sens de circulation allant du port d'entrée 21 vers les composants 23, 24, 25, 26.

Ainsi, on voit qu'une vanne 23, préférentiellement manuelle, est agencée sur la ligne d'admission 22 immédiatement en aval du port d'entrée 21. La vanne 23 est manœuvrable, préférentiellement par actionnement de la part de l'utilisateur, entre une position d'ouverture et une position de fermeture, et inversement, via un organe de manœuvre, tel un bouton rotatif (non montré), de manière à contrôler la circulation de gaz dans la ligne d'admission 22.

Lorsque la vanne 23 est en position de fermeture, la portion amont 22a de la ligne d'admission 22 située avant la vanne 23 (i.e. en amont) est soumise à la pression de détente du régulateur 31, alors que la portion intermédiaire 22b et la portion aval 22c situées après la vanne 23 (i.e. en aval) sont à pression atmosphérique.

En aval de la vanne 23, est agencé un régulateur de précision 24, qui est réglé sur une position fixe prédéfinie, c'est-à-dire qui est configuré pour délivrer une pression fixe donnée, comme décrit ci-après, de préférence comprise entre 350 et 700 mbar, par exemple de l'ordre de 500 mbar. On peut utiliser par exemple le régulateur de précision commercialisé par la société Beswick^{®} sous la référence commerciale PRD.

En aval de ce régulateur 24, un dispositif à orifice calibré 25 est agencé sur la ligne d'admission 22, ici dans sa portion aval 22c. On considère que le diamètre de ce dispositif à orifice calibré 25 est le diamètre de sortie, c'est-à-dire situé au niveau de sa sortie 25a. Le diamètre de sortie du dispositif à orifice calibré 25 est non-nul et préférentiellement inférieur à 500 µm.

On peut utiliser par exemple le dispositif à orifice calibré 25 commercialisé par la société O'Keefe Control^{®}, sous la référence commerciale BLP-2^{®}, qui présente un diamètre de sortie de l'ordre de 65 µm.

Le dispositif à orifice calibré 25 est couplé mécaniquement et relié fluidiquement à un dispositif à venturi 26 via un épaulement 261 du dispositif à venturi 26 venant se solidariser à la surface externe 25b du dispositif à orifice calibré 25, par exemple par vissage, montage en force ou tout autre technique.

Le dispositif à venturi 26 comprend un corps principal 260 définissant un volume interne 260a. Un col 262 relie l'épaulement 261 au corps principal 260.

De plus, le dispositif à venturi 26, en particulier le col 262, comprend un orifice d'admission de gaz 263 en communication fluidique avec l'atmosphère. L'orifice d'admission 263 peut revêtir différentes formes, telle que rectangulaire, circulaire, ou autre. De préférence, il présente une aire de section comprise entre 0.5 et 10 mm², par exemple de l'ordre de 5 mm² environ.

Le volume interne du col 262 du dispositif à venturi 26, qui est compris sensiblement entre la sortie 25a du dispositif à orifice calibré 25 et l'orifice d'admission 263, forme une chambre de venturi 264.

La sortie 25a du dispositif à orifice calibré 25 est en communication fluidique avec le volume interne 260a du corps principal 260 du dispositif à venturi 26, via le col 262.

Le corps principal 260 du dispositif à venturi 26 présente par ailleurs un conduit d'échappement 265 avec un orifice d'échappement 265a relié fluidiquement à l'atmosphère ambiante A et un orifice ou port de sortie 269 qui peut être relié fluidiquement à un dispositif de délivrance de NO 1, comme expliqué ci-après.

L'orifice ou port d'admission de gaz 263, l'orifice d'échappement 265a et le port de sortie 269 sont en communication fluidique avec le volume interne 260a du corps principal 260 du dispositif à venturi 26.

Tous les éléments formant le système de calibration 2 selon l'invention peuvent être insérés dans un boitier rigide 200, uniquement montré en pointillés, permettant d'assurer leur intégrité mécanique sans affecter les performances de l'ensemble.

Ci-après, on considère que la vanne 23 est manuelle et actionnable par l'utilisateur. Elle est appelée « vanne manuelle 23 ».

Lorsque l'utilisateur manœuvre la vanne manuelle 23 pour la passer en position d'ouverture, la pression régnant dans la portion amont 22a de la ligne d'admission 22 se propage en aval de la vanne manuelle 23, jusqu'au régulateur de précision 24. Le régulateur de précision 24 génère alors une pression de détente utile inférieure à la pression de détente fixée par le régulateur 32, de préférence comprise entre 350 et 700 mbar, par exemple de l'ordre de 500 mbar. La pression de détente utile se propage alors dans la portion aval 22c de la ligne d'admission 22, en amont de l'orifice calibré 25.

Or, il existe une relation entre la pression en amont du dispositif à orifice calibré 25 et le débit sortant dudit dispositif à orifice calibré 25 via son diamètre de sortie au niveau de sa sortie 25a.

Du fait de la faible dimension (i.e. < 500 µm) du diamètre de sortie de la sortie 25a, par exemple de l'ordre de 65 µm, l'orifice calibré 25 génère un fluide à un débit volumique faible (par exemple de l'ordre de 0.05 L/min) et à haute vélocité (par exemple de l'ordre de 3 m/s) en la sortie 25a qui va créer une dépression dans la chambre venturi 264, laquelle va elle-même créer un différentiel de pression négatif entre la pression absolue régnant dans la chambre venturi 264, et la pression absolue, i.e. c'est-à-dire la pression atmosphérique, de l'air ambiant A en communication fluidique avec la chambre venturi 264 via l'orifice d'admission 263.

Le différentiel de pression négatif créé engendre une aspiration d'un débit d'air provenant de l'atmosphère ambiante A via l'orifice d'admission 263. L'air aspiré contient notamment 20.9 vol.% d'O₂ (i.e. environ 21 % d'O₂) des quantités négligeables de NO et NO₂, i.e. de l'ordre de 0.05 ppmv, et bien entendu de l'azote et de l'argon, ou d'autres impuretés négligeables comme de la vapeur d'eau.

Le débit d'air entrant par l'orifice d'admission venturi 263 se mélange au débit de mélange NO/N₂ délivré par l'orifice calibré 25, i.e. via sa sortie 25a. Ici, le mélange gazeux NO/N₂ provenant de la bouteille de gaz 31, contient du NO à une concentration de 800 ppmv, le reste étant de l'azote (N₂).

En dimensionnant correctement le diamètre de sortie (au niveau de la sortie 25a) de l'orifice calibré 25 et l'orifice d'admission venturi 263, on peut obtenir, sur une plage de pression donnée, c'est-à-dire de pression en amont de l'orifice calibré 25, i.e. dans la portion aval 22c de la ligne d'admission 22, un rapport constant entre le débit sortant de l'orifice calibré 25 et le débit entrant par l'orifice d'admission venturi 263. Ce rapport peut être compris entre 10 et 30, par exemple de l'ordre de 19.

Ainsi, si le débit généré par l'orifice calibré 25 contient 800 ppmv de NO, et le de débit entrant par l'orifice d'admission venturi 263, une quantité négligeable de NO (e.g. environ 0.05 ppmv), le mélange des deux flux gazeux contient une concentration en NO de l'ordre de 40 ppmv, i.e. du fait du rapport ici de 19.

Le mélange gazeux à 40 ppm de NO se propage alors dans le volume interne 260a du corps principal 260 du dispositif à venturi 26 pour ensuite s'échapper à l'atmosphère ambiant A, via le conduit d'échappement 265 et le port de sortie 269.

Fig. 2 est un tableau de résultats illustrant des tests réalisés avec le dispositif de calibration 2 de Fig. 1, pour des valeurs de pression de détente utile comprises entre 350 et 2800 mbar.

Pour chaque pression de détente utile, le débit MFM1 (en I/min) provenant de la source de NO 3 et passant par l'orifice calibré 25, et le débit MFM2 (en I/min) entrant via l'orifice d'admission 263 du dispositif à venturi 26 ont été mesurés et les valeurs obtenues ont permis de déterminer le rapport (Ratio) entre les deux débits (i.e. rapport entre le débit entrant via l'orifice d'admission 263 au débit généré par l'orifice calibré 25).

On voit que le rapport a une valeur constante d'environ 19, sur une plage restreinte de pression, à savoir ici entre 350 et 700 mb. Au-delà, le rapport décroit au fur et à mesure que la pression de détente utile augmente. Ainsi, le ratio n'est plus que 12.25 pour une pression de 2800 mb. Ces résultats reflètent bien le rendement de tout dispositif à venturi qui atteint un maximum sur une plage de pression large, est maintenu sur une plage étroite au sein de la plage de pression large, pour ensuite diminuer au fur et à mesure que la pression augmente.

Partant de là, au vu de cette évolution du rapport (ratio) entre les deux débits et pour un mélange NO/N₂ à 800 ppmv de NO provenant de la source de NO 3, comme décrit ci-avant, la concentration en NO résultant du mélange des deux débits va être d'environ 40 ppmv sur la plage (350 mbar - 700 mbar), pour ensuite augmenter progressivement au fur et à mesure que la pression de détente utile augmente.

Ainsi, on détermine par exemple que la teneur en NO est de 43.36 ppmv à 1400 mbar et de 60.36 ppmv à 2800 mbar, comme consigné dans le tableau de Fig. 2.

Le rapport de dilution souhaité, par exemple ici 19, à son rendement maximal pour lequel une stabilité est obtenue sur une plage de pression donnée, peut être obtenu en dimensionnement spécifiquement le dispositif venturi 26, en particulier l'orifice d'admission 263. Ceci peut être fait par exemple via des tests empiriques de dimensionnement.

Au vu des résultats de Fig. 2, on préfère régler le régulateur de précision 24 à environ 500 mb. En effet, la pression de détente utile générée par le régulateur de précision 24 peut varier ou dériver un peu au cours du temps. Il est donc judicieux de fixer la valeur de détente du régulateur de précision 24 vers le milieu de la plage étroite 350-700 mbar afin de limiter le risque que le rapport de 19 ne soit plus respecté. En effet, si une dérive du régulateur de précision 24 se produit, par exemple si la pression qu'il délivre dérive légèrement au-dessus ou en-dessous de 500 mbar, le rapport de dilution souhaité reste égal à environ 19, c'est-à-dire à son rendement maximal. Ceci permet d'assurer une stabilité du rapport de dilution, y compris en cas de légère variation ou dérive de la pression de détente utile, et donc de maintenir ici une concentration en NO de l'ordre de 40 ppmv, après dilution par l'air ambiant amené par l'orifice d'admission 263 du dispositif à venturi 26.

Comme détaillé ci-après (cf. Fig. 4), le dispositif de calibration 2 de Fig. 1 peut être utilisé pour calibrer les capteurs d'un appareil de délivrance de NO 1, tel celui illustré sur Fig. 3, faisant partie d'une installation de fourniture de gaz 1000 à un patient comprenant ledit appareil de délivrance de NO 1 coopérant avec un ventilateur médical 300 et à un circuit patient 403, de manière à injecter du NO dans le circuit patient 403 qui véhicule en outre un gaz respiratoire contenant environ au moins 21%vol. d'oxygène typiquement de l'air ou un mélange O₂/N₂, fourni par le ventilateur médical 300.

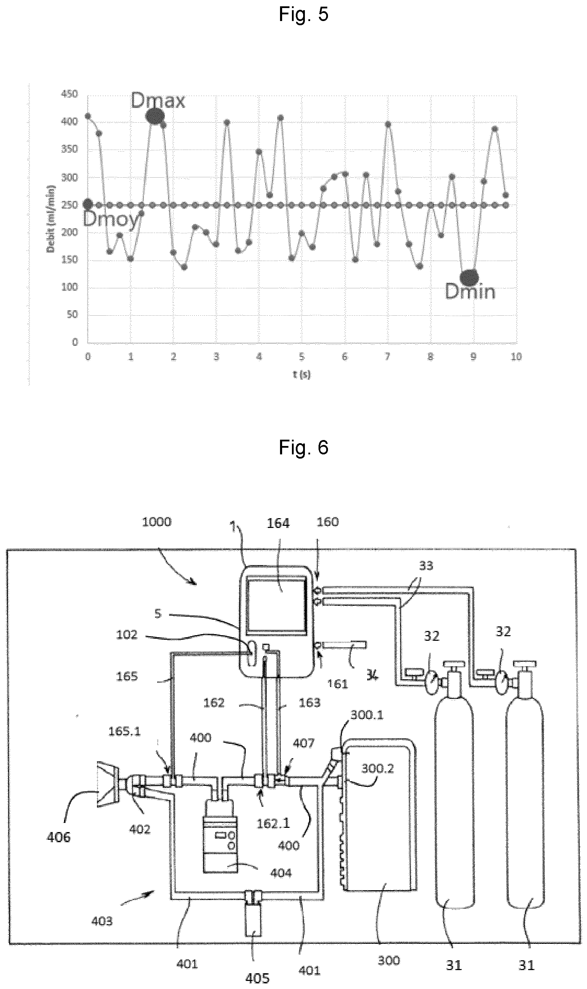

Un mode de réalisation d'une telle installation de fourniture de gaz 1000 est illustré sur Fig. 6. Elle comprend ici deux bouteilles de gaz sous pression 31 contenant chacune un mélange gazeux NO/N₂, à savoir ici un mélange gazeux NO/N₂ contenant 800 ppm vol. de NO (reste N₂), qui alimentent en mélange NO/N₂, le dispositif de délivrance de NO 1.

Les bouteilles de gaz 31 sont reliées fluidiquement au dispositif de délivrance de NO 1, via des lignes d'amenée de gaz 33, tel que des tuyaux ou conduits flexibles ou analogues, qui peuvent être équipées de dispositifs de régulation et/ou de suivi de la pression de gaz, tels que détendeur de gaz 32, manomètres...

Les lignes d'amenée de gaz 30 sont reliées à une ou plusieurs entrées de gaz 160 du dispositif de délivrance de NO 1 qui alimentent un passage de gaz interne servant à acheminer le gaz au sein du dispositif de délivrance de NO 1, c'est-à-dire dans le boitier 5 ou la carcasse externe du dispositif de délivrance de NO 1.

Le dispositif de délivrance de NO 1 comprend aussi une entrée d'oxygène 161 reliée fluidiquement, via une ligne d'amenée d'oxygène 34, tel un tuyau flexible ou analogue, à une source d'oxygène par exemple une bouteille d'oxygène sous pression ou un réseau hospitalier, c'est-à-dire une canalisation d'alimentation en oxygène agencée dans un bâtiment hospitalier.

L'installation de fourniture de gaz 10000 comprend par ailleurs un ventilateur médical 300, c'est-à-dire un appareil d'assistance respiratoire, qui fournit un flux de gaz respiratoire contenant au moins environ 21% d'oxygène, tel de l'air ou un mélange oxygène/azote (N₂/O₂).

Le ventilateur médical 23 et le dispositif de délivrance de NO 1 de l'installation de fourniture de gaz 100 sont en communication fluidique avec une ligne d'alimentation en gaz 400, aussi appelé circuit patient, servant à acheminer un flux gazeux vers le patient, lequel est formé par mélange du flux provenant du ventilateur médical 300 et du flux contenant le NO, i.e. le mélange gazeux NO/N₂, délivré par le dispositif de délivrance de NO 1.

Comme déjà expliqué, le dispositif de délivrance de NO délivre ou injecte le mélange NO/N₂, ici à 800 ppmv de NO, dans la ligne d'alimentation en gaz 400, via un conduit ou ligne d'injection 162, de manière à injecter (en 162.1) le flux de NO/N₂ dans le flux d'air ou de mélange oxygène/azote délivré par le ventilateur médical 300 et véhiculé par la ligne d'alimentation 400.

La ligne d'alimentation en gaz 22 comprend en outre un humidificateur de gaz 404 agencé en aval du site (162.1) où se fait l'injection de NO dans la ligne d'alimentation 22. Il permet d'humidifier le flux de gaz, e.g. mélange NO/N₂/air, avant qu'il ne soit inhalé par le patient à traiter, au moyen d'une interface respiratoire 406, telle une sonde d'intubation trachéale, un masque respiratoire ou analogue.

Est prévue aussi une ligne de récupération 401 des gaz expirés par le patient. La ligne d'alimentation en gaz 400 et la ligne de récupération 401 des gaz expirés sont raccordées à une pièce de raccordement 402, de préférence une pièce en Y, et définissent ainsi un circuit patient 403. La ligne d'alimentation en gaz 400 forme la branche inspiratoire du circuit patient 403, alors que la ligne de récupération 401 forme la branche expiratoire du circuit patient 403.

La ligne d'alimentation 400 en gaz est raccordée fluidiquement à un port de sortie 300.1 du ventilateur médical de manière à récupérer et acheminer le gaz, typiquement de l'air (ou mélange N₂/O₂ contenant environ 21% d'O₂) délivré par le ventilateur médical 300, alors que la ligne de récupération 401 des gaz expirés est raccordée fluidiquement à un port d'entrée 300.2 du ventilateur médical 300 de manière à retourner au ventilateur médical 300 tout ou partie du débit des gaz expirés par le patient.

La ligne de récupération 401 des gaz expirés peut comprendre un ou plusieurs composants optionnels, comme par exemple un dispositif d'élimination du CO₂ 405, i.e. un piège à CO₂, tel un bac à chaud ou autre, permettant d'éliminer le CO₂ présent dans les gaz expirés par le patient ou un filtre ou autre. La ligne de récupération 401 peut être utilisée par le ventilateur 300 pour détecter une fuite de gaz dans le circuit patient 403.

Un capteur de débit 407, par exemple du type à fil chaud ou à différentiel de pression, est agencé sur la ligne d'alimentation 400 en gaz, entre le ventilateur 300 et l'humidificateur 404, et est relié au dispositif de délivrance de NO 1, via une ligne de mesure de débit 163. Cet agencement set à mesurer le débit de gaz délivré par le ventilateur 300, tel de l'air ou un mélange N₂/O₂, et circulant dans la ligne d'alimentation 400, en amont du site de 162.1 raccordement du conduit ou ligne d'injection 162 où se fait le mélange NO/N₂/air. Ceci permet de mieux réguler la délivrance du flux de NO par le dispositif de délivrance de NO 1.

Comme détaillé ci-après, une ligne de prélèvement de gaz 165 relie fluidiquement la ligne d'alimentation en gaz 400 au dispositif de délivrance de NO 1.

La ligne de prélèvement de gaz 165 vient se raccorder fluidiquement (en 165.1) à la ligne d'alimentation en gaz 400, entre l'humidificateur 404 et la pièce de jonction 402, i.e. pièce en Y, typiquement à proximité immédiate de la pièce de jonction 402, et par ailleurs à un port d'entrée 102 du dispositif de délivrance de NO 1, par exemple un port 102 porté par un connecteur, raccord ou analogue permettant le raccordement de la ligne de prélèvement de gaz 165, tel un tuyau flexible ou analogue.

La ligne de prélèvement de gaz 165 permet de prélever dans la ligne d'alimentation en gaz 400 du circuit patient 403 des échantillons de gaz dont il convient de vérifier la conformité, et de les convoyer jusqu'au dispositif de délivrance de NO 1 où ils sont analysés dans un analyseur de gaz 10 interne, comme détaillé ci-après.

En particulier, il convient de vérifier que leur composition est conforme à celle du mélange gazeux NO/O₂/N₂ souhaité devant être administré au patient, notamment pour s'assurer qu'il ne contient pas de quantité excessive d'espèces NO₂ toxiques, que sa teneur en oxygène n'est pas hypoxique et que sa teneur en NO correspond à la posologie souhaitée.

Cette vérification de conformité se fait classiquement au moyen de moyens de mesure dédiés, typiquement des capteurs de NO₂, de NO et d'O₂, qui doivent eux-mêmes être calibrés périodiquement, par exemple toutes les semaines.

Ainsi, Fig. 3 schématise un mode de réalisation de l'appareil de délivrance de NO 1 auquel peut être raccordé, comme illustré en Fig. 4, le dispositif de calibration 2 de Fig. 1 afin de permettre de réaliser une calibration des moyens de mesure utilisés pour opérer la vérification de conformité du mélange gazeux, typiquement des capteurs de NO₂, de NO et d'O₂.

Cet appareil de délivrance de NO 1 comprend, de manière classique, un boitier 13 rigide, par exemple en polymère, traversé par un passage de gaz interne (non visible), tel un conduit de gaz ou analogue, pour acheminer le flux de NO/N₂ amené par la (ou les) ligne d'amenée de gaz 33 qui est alimentée par les bouteilles de mélange de NO/N₂ 31. Le passage de gaz interne relie fluidiquement l'entrée (ou les entrées) de gaz 160 (cf. Fig. 6) du dispositif de délivrance de NO 1 à la ligne d'injection 162 de manière à convoyer le flux gazeux à base de NO entre eux.

De façon classique, des moyens à valve (non montré), i.e. un (ou des) dispositif à valve, par exemple une pluralité d'électrovannes agencées en parallèle ou une ou des (électro)vannes proportionnelles, sont agencés sur le passage de gaz interne pour contrôler le flux gazeux qui y circule en direction de la ligne d'injection 162.

Les moyens à valve sont commandés par des moyens de pilotage 15, i.e. un (ou des) dispositif de pilotage, agencés dans le boitier, typiquement une carte électronique comprenant un (ou plusieurs) microprocesseur(s), typiquement un (ou des) microcontrôleur, mettant en œuvre un ou des algorithmes.

Les moyens de pilotage 15 permettent notamment d'ajuster ou contrôler le débit de gaz en pilotant les moyens à valve, typiquement d'ouvrir ou fermer cette ou ces vannes, pour obtenir un débit de gaz déterminé et/ou calculé par les moyens de pilotage 15 à partir d'une valeur réglée/fixée par l'utilisateur, et en fonction du débit de gaz, i.e. air, délivré par le ventilateur 300 et mesuré par le capteur de débit 407 agencé sur la ligne d'alimentation 400 en gaz et est relié au dispositif de délivrance de NO 1, par la ligne de mesure de débit 163, comme expliqué ci-avant. Les mesures de débit du flux délivré par le ventilateur 300 et circulant dans la ligne 400 sont fournies aux moyens de pilotage 150.

Le passage de gaz interne peut aussi comprendre un (ou des) débitmètre (non représenté) agencé en amont et/ou en aval des moyens à valve, pour déterminer le débit de gaz à base de NO circulant dans le dispositif de délivrance de NO 1. Le débitmètre peut être du type à différentiel de pression, à fil chaud ou autre. Il coopère avec les moyens de pilotage 150 pour leur fournir, là encore, des mesures de débit du flux de NO/N₂.

Par ailleurs, le dispositif de délivrance de NO 1 comprend aussi une interface graphique utilisateur ou IGU comprenant un afficheur graphique 174, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant.

Les moyens de pilotage 15 comprenant par exemple une carte de commande électronique 150 et une unité de contrôle 151 à microprocesseur, typiquement un microcontrôleur ou analogue. Les moyens de pilotage 15 permettent de contrôler ou commander tous les éléments électromécaniques de l'appareil de délivrance de NO 1. Plus précisément, la carte de commande 150 intègre préférentiellement l'unité de contrôle 151 et est configurée pour commander et par ailleurs analyser les signaux provenant des différents composants de l'appareil de délivrance de NO 1, tels que pompe, capteurs...

L'alimentation électrique de l'appareil de délivrance de NO 1, en particulier des composants nécessitant du courant électrique pour fonctionner, tels les moyens de pilotage 15, l'afficheur graphique 164..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant.

Par ailleurs, comme déjà dit, l'appareil de délivrance de NO 1 comprend un analyseur de gaz 10 interne qui est utilisé lors des procédures de calibration. Dans le mode de réalisation de Fig. 3, tout ou partie des éléments de l'analyseur de gaz 10 peuvent être agencés dans le boitier 5, par exemple en polymère, formant l'enveloppe externe de l'appareil de délivrance de NO 1.

Plus précisément, l'analyseur de gaz 10 comprend un premier port d'entrée 100 et un second port d'entrée 102, typiquement situés à l'extérieur du boitier 5 de l'appareil de délivrance de NO, permettant d'alimenter une ligne d'analyse 110 de l'analyseur 10 avec du gaz, où :
- le premier port d'entrée 100 est relié fluidiquement à un premier port amont 104a d'une vanne 3:2 104, via une première ligne 101 ; et
- le second port d'entrée 102 est relié fluidiquement à un second port amont 104b de l'électrovanne 104, via une seconde ligne 102.

La vanne 3:2 104, de préférence une électrovanne, comprend en outre un port aval 104c relié fluidiquement à la ligne d'analyse 110 qui comprend des moyens de mesure 120-122. La ligne d'analyse 110 se termine par ailleurs par un port de sortie 110a relié fluidiquement à l'atmosphère ambiante A.

Ce type d'(électro)vanne 3:2 104 est disponible dans le commerce, par exemple auprès de la société **IMI** FAS^{®}.

Les moyens de pilotage 15 commandent l'(électro)vanne 3:2 104 de manière à réaliser, en fonction d'une configuration déterminée par les moyens de pilotage 15, une connexion fluidique entre le premier port amont 104a ou alternativement le second port amont 104b, et le port aval 104c, donc avec la ligne d'analyse 110.

La ligne d'analyse 110 comprend quant à elle des moyens de mesure de NO₂ 120, tel un capteur de NO₂, des moyens de mesure de NO 121, tel un capteur de NO 121, des moyens de mesure d'oxygène 122, tel un capteur d'O₂, et des moyens de mesure de débit 130, tel un capteur de débit. Ces moyens de mesure de NO₂ 120, de NO 121 et d'O₂ 122 sont de type électrochimiques. De tes capteurs sont disponibles auprès de la société Honeywell.

Par ailleurs, les moyens de mesure de débit 130 déterminant le débit circulant dans la ligne d'analyse sont ou comprennent préférentiellement un capteur massique, par exemple disponible auprès de la société Sensirion^{®}.

En outre, la ligne d'analyse 110 comprend un dispositif d'aspiration de gaz 140, telle une pompe ou analogue, préférentiellement une pompe à diaphragme, permettant de faire circuler un débit de gaz dans la ligne d'analyse 110 comme expliqué ci-après. Une pompe utilisable est disponible auprès de la société Parker^{®} ou de la société Thomas ^{®}.

Les moyens de pilotage 15 sont configurés pour récupérer et traiter, i.e. analyser, les signaux provenant des différents capteurs 120-121, 130 de l'analyseur de gaz 10, et d'agir en réponse à ces signaux, comme expliqué ci-après, notamment pour opérer une calibration des capteurs.

Ainsi, Fig. 4 schématise une association du dispositif de calibration 2 de Fig. 1 de l'invention à l'appareil de délivrance de NO 1 de Fig. 3 afin de permettre de réaliser une calibration des moyens de mesure de NO₂ 120-122, c'est-à-dire des capteurs de NO₂, de NO et d'O₂.

Pour lancer une procédure de calibration des moyens de mesure de NO₂ 120-122, l'utilisateur raccorde d'abord fluidiquement les moyens de raccordement aval 269a situés au niveau du port de sortie 269 du dispositif à venturi 26 au second port d'entrée 102 de l'analyseur de gaz 10, par exemple par vissage, par emboitement ou tout autre type de connexion pouvant assurer une connexion fluidique entre le volume interne 260a du corps principal 260 du dispositif à venturi et une ligne d'alimentation en gaz, i.e. appelée ci-après seconde ligne 103, de l'analyseur de gaz 10 de l'appareil de délivrance de NO 1 à calibrer.

La vanne manuelle 23 est laissée ou mise en position de fermeture, c'est-à-dire fermée, de sorte qu'aucun gaz sous pression ne puisse circuler vers la portion intermédiaire 22b et la portion aval 22c de la ligne d'admission 22.

L'utilisateur indique alors aux moyens de pilotage 15, par exemple via l'IGU de l'appareil de délivrance de NO 1, de lancer une séquence de calibration des moyens de mesure de NO₂ 120-122, i.e. des capteurs. Les moyens de pilotage 15 commandent alors l'électrovanne 104 pour mettre son port aval 104c en relation fluidique avec le premier port amont 104a, et par ailleurs les moyens d'aspiration 140, typiquement une pompe, pour aspirer de l'air ambiant A, à débit constant, via le premier port d'entrée 100 dans respectivement la première ligne 101 puis la ligne d'analyse de gaz 110, avant de rejeter cet air à l'atmosphère ambiante A via le port de sortie 110a.

Le débit d'air circulant dans la ligne d'analyse de gaz 110 est maintenu constant, par exemple égal à 250 ml/min environ, par les moyens de pilotage 15 grâce aux mesures de débit opérées par le capteur de débit 130, qui sont envoyées aux moyens de pilotage 15 et traitées au sein de ceux-ci. Les moyens de pilotage 15 ajustent donc en permanence la commande des moyens d'aspiration 140 pour obtenir le débit cible désiré par rapport aux mesures opérées.

Dans tous les cas, le flux gazeux circulant dans la ligne d'analyse de gaz 110 est de l'air ambiant, présentant des concentrations connues et sensiblement constantes de NO, NO₂ et O₂, à savoir une concentration en O₂ de l'ordre de 20.9% et des concentrations négligeables, i.e. quasi nulles, de NO et NO₂ (i.e. < 0.05 ppmv).

Ainsi, on peut réaliser un étalonnage ou calibration à « zéro » des capteurs de NO₂ 120 et NO 121, et un étalonnage « complet » du capteur O₂ 122 (i.e. 20.9% O₂).

Ensuite, l'utilisateur opère une ouverture de la vanne manuelle 23 pour fournir du mélange gazeux à base de NO, i.e. provenant de la source de NO 3 (i.e. 800 ppm NO ici), aux portions intermédiaires et aval 22b, 22c.

La portion aval 22c est à la pression de détente utile du fait du réglage du régulateur de précision 24 et il s'ensuit que, comme déjà expliqué, un mélange contenant ici 40 ppmv de NO remplit le volume interne 260a du corps principal 260 du dispositif à venturi 26.

A ce stade, l'électrovanne 104 relie toujours fluidiquement son premier port amont 104a à son port aval 104c de sorte que la seconde ligne 103 est isolée de la ligne d'analyse de gaz 110, i.e. le second port amont 104b de ladite électrovanne 104 est fermé. Ainsi, le port de sortie 269 du dispositif à venturi 26, qui est connecté au second port d'entrée 102, est lui-même isolé, c'est-à-dire que le mélange de gaz contenant 40 ppmv de NO présent dans le volume interne 260a du corps principal 260 ne peut emprunter le port de sortie 269. Ainsi, l'intégralité du débit gazeux, appelé débit utile, qui est égal à la somme du débit sortant de l'orifice calibré 25 par son diamètre de sortie 25a et du débit d'air entrant par l'orifice d'admission 263, va s'échapper à l'atmosphère ambiante A via le conduit d'échappement 265.

Une fois la vanne manuelle 23 en position d'ouverture, et préférentiellement confirmation de cette position d'ouverture par l'utilisateur via l'IGU, les moyens de pilotage 15 commande l'électrovanne 104 pour relier fluidiquement le second port amont 104b au port aval 104c. Ainsi, la seconde ligne 103 devient fluidiquement connectée à la ligne d'analyse de gaz 110.

Du fait de la connexion du dispositif de calibration 2 de l'invention au second port d'entrée 102 de l'appareil de délivrance de NO 1, le volume interne 260a du corps principal 260 est alors en relation fluidique, via le port de sortie 269 du dispositif à venturi 26, à la ligne d'analyse de gaz 110.

La pompe 140, qui est toujours contrôlée pour prélever un débit de l'ordre de 250 ml/min, va alors aspirer une partie du débit utile (i.e. somme du débit sortant de l'orifice calibré 25 par son diamètre de sortie 25a et du débit entrant par l'orifice d'admission venturi 263), donc exposer les moyens de mesure 120-122, à savoir les capteurs de NO₂, NO et O₂, à un mélange gazeux contenant 40 ppmv de NO.

Fig. 5 schématise les performances standard d'une pompe à diaphragme utilisée en tant que dispositif d'aspiration de gaz 140 au sein de la ligne d'analyse de gaz 110 de l'appareil de délivrance de NO 1 de Fig. 4.

Plus précisément, la courbe de débit en fonction du temps de Fig. 5 montre qu'il existe des fluctuations rapides de débit transitant dans la ligne d'analyse de gaz 110, qui sont mesurées par le capteur de débit 130.

Si la valeur moyenne de débit Dmoy, par exemple calculée sur 10 secondes, est, comme attendu, d'environ 250 ml/min, le débit instantané oscille entre une valeur maximale de débit Dmax de 400 ml/min environ et une valeur minimale de débit Dmin de 125 ml/min environ.

Ceci impose au dispositif à venturi 26 de délivrer un débit utile supérieur à la valeur maximale de débit Dmax mesurée par le capteur de débit 130. En effet, si ce débit utile est inférieur au débit maximal Dmax, alors tout débit demandé par la pompe 140 qui est supérieur au débit utile sera complété par l'admission d'air ambient A via le conduit d'échappement 265 du dispositif à venturi 26. Or, cette admission non désirée par le conduit d'échappement 265 se traduira alors par une dilution du mélange présent dans le volume interne 260a du corps principal 260 dispositif à venturi 26, et donc par une diminution de la concentration en NO souhaitée (qui devrait être ici à égale à 40 ppmv).

En se référant à Fig. 2, on note que le débit utile, qui est la somme du débit sortant de l'orifice calibré 25 par son diamètre de sortie 25a et du débit entrant par l'orifice d'admission venturi 263, est de 0.73 l/min (730 ml/min) pour une pression de détente utile de 350 mb, et de 1.06 l/min (1060 ml/min) pour une pression de détente utile de 750 mb. En réglant le régulateur de précision à 500 mb, le débit utile sera bien supérieur au débit maximal Dmax demandé par la pompe.

En d'autres termes, la somme du débit sortant de l'orifice calibré 25 par son diamètre de sortie 25a et le débit entrant par l'orifice d'admission venturi 263 sera toujours supérieur à la demande instantanée de la pompe 140 et ainsi l'excédent de gaz, i.e. de mélange, empruntera le conduit d'échappement 265 de l'élément venturi 26 pour être rejeté à l'atmosphère ambiante

Il s'ensuit que le gaz empruntant le port de sorte 269 du dispositif à venturi 26, le second port d'entrée 102, la seconde ligne 103 et la ligne d'analyse de gaz 110 a la concentration désirée en NO, à savoir ici 40 ppmv.

Ce mélange à la concentration désirée va alors exposer le capteur de NO 121 à la concentration cible.

Après une phase de stabilisation, les moyens de pilotage 15 peuvent déterminer le point de calibration haut à 40 ppmv du capteur de NO 121.

Les moyens de pilotage 15 peuvent alors arrêter la pompe 140 de manière à ce que plus aucun gaz ne circule dans la ligne d'analyse de gaz 110. Les moyens de mesure 120, 121, 122 sont dès lors exposés à un gaz de concentration initiale égale à 40 ppmv en NO et comprenant par ailleurs une teneur en O₂ de l'ordre de 20.9% vol.

Or, il est connu qu'en présence d'oxygène, le NO s'oxyde progressivement en NO₂ en fonction notamment de leurs teneurs respectives et de leur temps de contact.

Par le biais d'un modèle établi, sous forme d'équation reliant une concentration en NO₂ à un temps donné en fonction des teneurs en NO et en O₂ initiales, les moyens de pilotage 15 laissent une réaction de transformation du NO en NO₂ s'opérer pendant une durée donnée, par exemple pendant 8 minutes, jusqu'à formation d'une teneur donnée en NO₂, par exemple jusqu'à formation de 5 ppmv de NO₂. Cette teneur en NO₂ sert alors de point de calibration haut du capteur de NO₂ 120.

Les moyens de mesure 120-12, en particulier les capteurs de NO₂ 120, de NO 121 et d'O₂ 122, sont alors parfaitement étalonnés.

Les moyens de pilotage 15 peuvent alors initier une dernière étape consistant à commander l'électrovanne 104 de manière à relier fluidiquement son premier port amont 104a à son port aval 104c et de piloter la pompe 140 pour réguler un débit, mesuré par le capteur de débit 130, de l'ordre de 250 ml/min, comme décrit précédemment et ce, afin de faire circuler de l'air ambiant A dans la ligne d'analyse de gaz 110 pour la « purger » des gaz, notamment du NO qui peut s'y trouver.

Dans le même temps, les moyens de pilotage 15 peuvent préférentiellement informer l'utilisateur que la procédure de calibration a été réalisée pour l'inciter à fermer la vanne manuelle 23 afin de stopper la génération d'un mélange de gaz à 40 ppmv par le dispositif à venturi 26.

Le dispositif de calibration 2 de l'invention peut alors être désolidarisé de la seconde prise 102 de l'appareil de délivrance de NO 1 et rangé, dans l'attente d'une nouvelle procédure de calibration.

Le dispositif de calibration 2 de l'invention permet de simplifier la procédure de calibration régulière des capteurs de l'analyseur de gaz 10 de l'appareil de délivrance de NO 1, en particulier le capteur de NO, le capteur de NO₂ et le capteur d'O₂.

## Revendications

1. Dispositif de calibration (2) pour calibrer les moyens de mesure (120-122) d'un appareil de délivrance de NO (1), lesdits moyens de mesure (120-122) comprenant un capteur de NO, un capteur de NO₂ et un capteur d'O₂, ledit dispositif de calibration (2) comprenant une ligne d'admission de gaz (22) comprenant, agencés en série :
- une vanne (23) pour contrôler la circulation de gaz dans la ligne d'admission de gaz (22),
- un régulateur de précision (24) configuré pour délivrer une pression fixe prédéfinie dans la ligne d'admission de gaz (22),
- un dispositif à orifice calibré (25) et
- un dispositif à venturi (26) comprenant un corps principal (260) définissant un volume interne (260a) et comprenant :
▪ un orifice d'entrée de gaz relié fluidiquement au dispositif à orifice calibré (25) pour permettre une entrée de gaz provenant du dispositif à orifice calibré (25), dans le volume interne (260a),
▪ un orifice d'admission de gaz (263) relié fluidiquement à l'atmosphère (A) pour permettre une entrée d'air dans le volume interne (260a),
▪ un orifice d'échappement (265a) relié fluidiquement à l'atmosphère (A) pour permettre une évacuation à l'atmosphère (A), d'une partie du gaz contenu dans le volume interne (260a), et
▪ un port de sortie (269) pour fournir au moins une partie du gaz contenu dans le volume interne (260a) à un appareil de délivrance de NO (1).

2. Dispositif selon la revendication 1, **caractérisé en ce que** la vanne (23) est mobile entre au moins :
- une position de fermeture dans laquelle la circulation de gaz dans la ligne d'admission de gaz (22) est empêchée et
- une position d'ouverture dans laquelle la circulation de gaz dans la ligne d'admission de gaz (22) est autorisée.

3. Dispositif selon la revendication 1, **caractérisé en ce que** le régulateur de précision (24) est configuré pour délivrer une pression fixe comprise entre 350 et 700 mbar.

4. Dispositif selon la revendication 1, **caractérisé en ce que** le dispositif à orifice calibré (25) comprend un orifice de sortie (25a) de diamètre inférieur à 500 µm

5. Dispositif selon la revendication 1, **caractérisé en ce que** l'orifice d'admission de gaz (263) présente une aire de section comprise entre 0.5 et 10 mm².

6. Dispositif selon la revendication 1, **caractérisé en ce que** la ligne d'admission de gaz (22), la vanne (23), le régulateur de précision (24), le dispositif à orifice calibré (25) et le dispositif à venturi (26) sont agencés dans un boitier commun (200).

7. Dispositif selon l'une des revendications 1 ou 2, **caractérisé en ce que** la vanne (23) est une vanne manuelle actionnable par un utilisateur.

8. Dispositif selon la revendication 1, **caractérisé en ce qu'**il comprend en outre des moyens de raccordements amont (20) agencés au niveau d'un port d'entrée (21) de la ligne d'admission de gaz (22), configurés pour y raccorder un conduit flexible (33).

9. Dispositif selon l'une des revendications 1 ou 8, **caractérisé en ce qu'**il comprend en outre des moyens de raccordement aval (269a) agencés au niveau du port de sortie (269) du dispositif à venturi (26), configurés pour y raccorder une ligne d'alimentation en gaz (103) d'un analyseur de gaz (10) d'un appareil de délivrance de NO (1).

10. Dispositif selon la revendication 1, **caractérisé en ce que** le diamètre de sortie de l'orifice calibré (25) et le diamètre de l'orifice d'admission **j^{u}** de gaz (263) du dispositif à venturi (26) sont dimensionnés pour obtenir un rapport constant entre le débit sortant de l'orifice calibré (25) et le débit entrant par l'orifice d'admission de gaz (263) du dispositif à venturi (26) compris entre 10 et 30.

11. Utilisation d'un dispositif de calibration (2) selon l'une des revendications précédentes, pour calibrer les moyens de mesure (120-122) d'un appareil de délivrance de NO (1), lesdits moyens de mesure (120-122) comprenant un capteur de NO, un capteur de NO₂ et un capteur d'O₂.

12. Utilisation selon la revendication 11, **caractérisée en ce que** le dispositif de calibration (2) est raccordé fluidiquement à une bouteille de gaz (3, 31) contenant un mélange NO/N₂ alimentant la ligne d'admission de gaz (22) du dispositif de calibration (2), en amont de la vanne (23).

## Patentansprüche

1. Kalibriervorrichtung (2) zum Kalibrieren der Messmittel (120-122) eines Gerätes zur NO-Abgabe (1), wobei die Messmittel (120-122) einen NO-Sensor, einen NO₂-Sensor und einen O₂-Sensor umfassen, wobei die Kalibriervorrichtung (2) eine Gaseinlassleitung (22) umfasst, welche umfasst, in Reihe angeordnet:
- ein Ventil (23) zum Steuern des Strömens von Gas in der Gaseinlassleitung (22),
- einen Präzisionsregler (24), der dafür ausgelegt ist, einen vordefinierten festen Druck in der Gaseinlassleitung (22) zu liefern,
- eine Vorrichtung mit kalibrierter Öffnung (25) und
- eine Venturi-Vorrichtung (26), die einen Hauptkörper (260) umfasst, der ein Innenvolumen (260a) definiert und umfasst:
• eine Gaseintrittsöffnung, die mit der Vorrichtung mit kalibrierter Öffnung (25) in Fluidverbindung steht, um einen Eintritt von Gas, das von der Vorrichtung mit kalibrierter Öffnung (25) kommt, in das Innenvolumen (260a) zu ermöglichen,
• eine Gaseinlassöffnung (263), die mit der Atmosphäre (A) in Fluidverbindung steht, um einen Eintritt vom Luft in das Innenvolumen (260a) zu ermöglichen,
• eine Ausströmöffnung (265a), die mit der Atmosphäre (A) in Fluidverbindung steht, um ein Ablassen eines Teils des in dem Innenvolumen (260a) enthaltenen Gases in die Atmosphäre (A) zu ermöglichen, und
• einen Austrittsanschluss (269), um wenigstens einen Teil des in dem Innenvolumen (260a) enthaltenen Gases einem Gerät zur NO-Abgabe (1) zuzuführen.

2. Vorrichtung nach Anspruch 1, **dadurch gekennzeichnet, dass** das Ventil (23) beweglich ist zwischen mindestens:
- einer Schließposition, in welcher das Strömen von Gas in der Gaseinlassleitung (22) verhindert wird, und
- einer Öffnungsposition, in welcher das Strömen von Gas in der Gaseinlassleitung (22) ermöglicht wird.

3. Vorrichtung nach Anspruch 1, **dadurch gekennzeichnet, dass** der Präzisionsregler (24) dafür ausgelegt ist, einen festen Druck zwischen 350 und 700 mbar zu liefern.

4. Vorrichtung nach Anspruch 1, **dadurch gekennzeichnet, dass** die Vorrichtung mit kalibrierter Öffnung (25) eine Austrittsöffnung (25a) mit einem Durchmesser von weniger als 500 µm umfasst.

5. Vorrichtung nach Anspruch 1, **dadurch gekennzeichnet, dass** die Gaseinlassöffnung (263) eine Querschnittsfläche zwischen 0,5 und 10 mm² aufweist.

6. Vorrichtung nach Anspruch 1, **dadurch gekennzeichnet, dass** die Gaseinlassleitung (22), das Ventil (23), der Präzisionsregler (24), die Vorrichtung mit kalibrierter Öffnung (25) und die Venturi-Vorrichtung (26) in einem gemeinsamen Gehäuse (200) angeordnet sind.

7. Vorrichtung nach einem der Ansprüche 1 oder 2, **dadurch gekennzeichnet, dass** das Ventil (23) ein handbetätigtes Ventil ist, das von einem Benutzer betätigbar ist.

8. Vorrichtung nach Anspruch 1, **dadurch gekennzeichnet, dass** sie außerdem stromaufwärtige Anschlussmittel (20), die an einem Eintrittsanschluss (21) der Gaseinlassleitung (22) angeordnet sind, umfasst, die dafür ausgelegt sind, hier eine flexible Leitung (33) anzuschließen.

9. Vorrichtung nach einem der Ansprüche 1 oder 8, **dadurch gekennzeichnet, dass** sie außerdem stromabwärtige Anschlussmittel (269a), die am Austrittsanschluss (269) der Venturi-Vorrichtung (26) angeordnet sind, umfasst, die dafür ausgelegt sind, hier eine Gasversorgungsleitung (103) eines Gasanalysators (10) eines Gerätes zur NO-Abgabe (1) anzuschließen.

10. Vorrichtung nach Anspruch 1, **dadurch gekennzeichnet, dass** der Austrittsdurchmesser der kalibrierten Öffnung (25) und der Durchmesser der Gaseinlassöffnung (263) der Venturi-Vorrichtung (26) dafür bemessen sind, ein konstantes Verhältnis zwischen der aus der kalibrierten Öffnung (25) austretenden Durchflussmenge und der über die Gaseinlassöffnung (263) der Venturi-Vorrichtung (26) eintretenden Durchflussmenge zu erhalten, das zwischen 10 und 30 liegt.

11. Verwendung einer Kalibriervorrichtung (2) nach einem der vorhergehenden Ansprüche zum Kalibrieren der Messmittel (120-122) eines Gerätes zur NO-Abgabe (1), wobei die Messmittel (120-122) einen NO-Sensor, einen NO₂-Sensor und einen O₂-Sensor umfassen.

12. Verwendung nach Anspruch 11, **dadurch gekennzeichnet, dass** die Kalibriervorrichtung (2) mit einer Gasflasche (3, 31) stromaufwärts des Ventils (23) in Fluidverbindung steht, die eine NO/N₂-Mischung enthält, welche die Gaseinlassleitung (22) der Kalibriervorrichtung (2) speist.

## Claims

1. Calibration device (2) for calibrating the measuring means (120-122) of an NO delivery apparatus (1), said measuring means (120-122) comprising an NO sensor, an NO₂ sensor and an O₂ sensor, said calibration device (2) comprising a gas intake line (22) comprising, arranged in series:
- a valve (23) for controlling the circulation of gas in the gas intake line (22),
- a precision regulator (24) configured to deliver a predefined fixed pressure in the gas intake line (22),
- a calibrated orifice device (25), and
- a venturi device (26) comprising a main body (260) defining an internal volume (260a) and comprising:
• a gas inlet orifice fluidically connected to the calibrated orifice device (25) in order to allow gas from the calibrated orifice device (25) to enter the internal volume (260a),
• a gas intake orifice (263) fluidically connected to the atmosphere (A) in order to allow air to enter the internal volume (260a),
• an exhaust orifice (265a) fluidically connected to the atmosphere (A), for discharging some of the gas contained in the internal volume (260a) to the atmosphere (A), and
• an outlet port (269) for supplying at least some of the gas contained in the internal volume (260a) to an NO delivery apparatus (1).

2. Device according to Claim 1, **characterized in that** the valve (23) is movable between at least:
- a closed position, in which the circulation of gas in the gas intake line (22) is prevented, and
- an open position, in which the circulation of gas in the gas intake line (22) is permitted.

3. Device according to Claim 1, **characterized in that** the precision regulator (24) is configured to deliver a fixed pressure of between 350 and 700 mbar.

4. Device according to Claim 1, **characterized in that** the calibrated orifice device (25) comprises an outlet orifice (25a) with a diameter of less than 500 µm.

5. Device according to Claim 1, **characterized in that** the gas intake orifice (263) has a cross-sectional area of between 0.5 and 10 mm².

6. Device according to Claim 1, **characterized in that** the gas intake line (22), the valve (23), the precision regulator (24), the calibrated orifice device (25) and the venturi device (26) are arranged in a common housing (200).

7. Device according to either of Claims 1 and 2, **characterized in that** the valve (23) is a manual valve actuatable by a user.

8. Device according to Claim 1, **characterized in that** it further comprises upstream connection means (20) arranged at an inlet port (21) of the gas intake line (22) and configured to connect a flexible conduit (33) thereto.

9. Device according to either of Claims 1 and 8, **characterized in that** it further comprises downstream connection means (269a) arranged at the outlet port (269) of the venturi device (26) and configured to connect thereto a gas feed line (103) of a gas analyser (10) of an NO delivery apparatus (1).

10. Device according to Claim 1, **characterized in that** the outlet diameter of the calibrated orifice (25) and the diameter of the gas intake orifice (263) of the venturi device (26) are dimensioned to obtain a constant ratio, between the flow rate leaving the calibrated orifice (25) and the flow rate entering through the gas intake orifice (263) of the venturi device (26), of between 10 and 30.

11. Use of a calibration device (2) according to one of the preceding claims, for calibrating the measuring means (120-122) of an NO delivery apparatus (1), said measuring means (120-122) comprising an NO sensor, an NO₂ sensor and an O₂ sensor.

12. Use according to Claim 11, **characterized in that** the calibration device (2) is fluidically connected to a gas cylinder (3, 31) containing an NO/N₂ mixture feeding the gas intake line (22) of the calibration device (2), upstream of the valve (23).
